# EUROPEAN PATENT APPLICATION

(11) **EP 4 491 949 A1**
(43) Date of publication of application: **15.01.2025**
(21) Application number: 22958599.7
(22) Date of filing: 16.11.2022
(51) Int. Cl.: F24C 15/20, F24C 7/00

(54) **FUME EXTRACTING MECHANISM AND INDUCTION COOKER INTEGRATED STOVE**

(30) Priority: 13.09.2022 CN 202211107886; 13.09.2022 CN 202222421277 U
(71) Applicant: Guangdong Arcair Appliance Co., Ltd., Guangdong 528318 (CN)
(72) Inventor: KANG, Zuotian, Foshan, Guangdong 528318 (CN); TANG, Haijiang, Foshan, Guangdong 528318 (CN); LIANG, Haisheng, Foshan, Guangdong 528318 (CN)
(74) Representative: Ipey
(86) International application number: PCT/CN2022/132339
(87) International publication number: WO 2024/055411

(57) **Abstract**

The present disclosure provides a fume extracting mechanism and further provides an induction cooker integrated stove using the fume extracting mechanism. The fume extracting mechanism includes an air duct assembly, which includes a longitudinally-extending first flow channel and a transversely-extending second flow channel. The second flow channel is in communication with a lower part of the first flow channel. An air inlet is opened at an upper part of the first flow channel. A connection opening is opened on the second flow channel. The first flow channel and the second flow channel form a bending channel for air flow. An air inlet end in a middle part of a volute fan is connected with the connection opening, and the volute fan sucks air from the bending channel such that the air enters the air inlet and flows through the bending channel and then flows out of an exhaust opening of the volute fan. By using the L-shaped bending channel in cooperation with the volute fan, on the one hand, the characteristics of high air pressure, low rotation speed, low noise and high energy efficiency can be realized to achieve the effect of noise reduction and air suction improvement, and on the other hand, the entire structure is compact and the occupied space is small, making it more suitable for small mounting spaces.

## Description

### TECHNICAL FIELD

The present disclosure relates to fume extracting apparatuses and in particular to a fume extracting mechanism and an induction cooker integrated stove.

### BACKGROUND

The range hoods are usually mounted above countertop to upwardly suck fumes, and some range hoods are mounted on countertop to downwardly suck fumes. For those range hoods mounted on countertop on the current markets, a large-space-occupying ventilation duct is usually needed to cooperate with exhaust fans. In this case, the entire structure occupies a large space and cannot be applied to the environments with relatively small mounting spaces, and especially cannot meet the standard requirements of European region for kitchen cabinet mounting and placement. For this reason, it is necessary to make improvements.

### SUMMARY

The present disclosure aims to solve, at least to some degree, one of the above technical problems in the related arts. For this purpose, the present disclosure provides a fume extracting mechanism.

In order to achieve the above object, the present disclosure employs the following technical solution.

The present disclosure further provides an induction cooker integrated stove using the above fume extracting mechanism.

According to a first aspect of embodiments of the present disclosure, there is provided a fume extracting mechanism, which includes:
an air duct assembly, wherein the air duct assembly includes a longitudinally-extending first flow channel and a transversely-extending second flow channel, the second flow channel is in communication with a lower part of the first flow channel, an air inlet is opened at an upper part of the first flow channel, a connection opening is opened on the second flow channel, and the first flow channel and the second flow channel form a bending channel for air flow; and
a volute fan, wherein an air inlet end in a middle part of the volute fan is connected with the connection opening, and the volute fan sucks air from the bending channel such that the air enters the air inlet and flows through the bending channel and then flows out of an exhaust opening of the volute fan.

The fume extracting mechanism according to the embodiments of the present disclosure at least have the following beneficial effects: by using the L-shaped bending channel in cooperation with the volute fan, on the one hand, the characteristics of high air pressure, low rotation speed, low noise and high energy efficiency can be realized to achieve the effect of noise reduction and air suction improvement, and on the other hand, the entire structure is compact and the occupied space is small, making it more suitable for small mounting spaces.

According to some embodiments of the present disclosure, the first flow channel extends vertically, the second flow channel extends horizontally, and the first flow channel and the second flow channel form an L shape.

According to some embodiments of the present disclosure, the connection opening is opened on an upper end surface of the second flow channel, the volute fan is placed on the second flow channel, the air inlet end faces downward to connect with the connection opening, and the exhaust opening faces horizontally.

According to some embodiments of the present disclosure, the air inlet with an opening facing upward is opened on a top of the first flow channel.

According to some embodiments of the present disclosure, the air inlet is presented as flaring shape, and a diameter of the air inlet is greater than a diameter of the first flow channel.

According to some embodiments of the present disclosure, the air inlet is provided with an air guide member, and a bottom of the air guide member protrudes downward to form a V shape.

According to some embodiments of the present disclosure, the exhaust opening is provided with an air purifying device which includes an Ultraviolet (UV) lamp module and an ionization ozone generation module.

According to a second aspect of embodiments of the present disclosure, there is provided an induction cooker integrated stove, which includes a base body, an induction cooker and the above-mentioned fume extracting mechanism. The induction cooker is mounted on the base body, and the fume extracting mechanism is mounted inside the base body. An induced air pipe is disposed at a side of the base body. The exhaust opening is in communication with the induced air pipe. A bearing platform is disposed on an upper part of the base body, and the air inlet is connected to the bearing platform.

The induction cooker integrated stove according to the embodiments of the present disclosure at least have the following beneficial effects: since the volume of the entire structure of the induction cooker integrated stove is very small, the stove can adapt to multiple regions of the world and especially to the European region and comply with national standard requirements for kitchen cabinet mounting and placement. In this way, the defect that such products from the fellow companies are used only when the kitchen cabinet mounting size is customized can be overcome and the use requirements of the market users can be satisfied.

According to some embodiments of the present disclosure, the air inlet is higher than or flush with an upper end surface of the bearing platform, and the induction cooker is located below the bearing platform.

According to some embodiments of the present disclosure, one or more induction cookers are mounted on the base body, and the air inlet is connected at a middle position or a position of a side of the bearing platform.

Additional aspects and advantages of the present disclosure will be partially given in the following descriptions and partially become obvious from the following descriptions or understood by practice of the present disclosure.

### BRIEF DESCRIPTION OF DRAWINGS

The above and/or additional aspects and advantages of the present disclosure will become obvious and intelligible from the descriptions made to the embodiments by referring to the drawings.
FIG. 1 is a structural schematic diagram of a fume extracting mechanism.
FIG. 2 is an exploded view of a partial structure of FIG. 1.
FIG. 3 is another structural schematic diagram of the fume extracting mechanism.
FIG. 4 is a structural schematic diagram of an induction cooker integrated stove.
FIG. 5 is a schematic diagram of the induction cooker integrated stove according to an embodiment of the present disclosure.
FIG. 6 is a schematic diagram of the induction cooker integrated stove according to another embodiment of the present disclosure.

Numerals of the drawings are described below:
100. air duct assembly, 110. first flow channel, 111.air inlet, 120. second flow channel, 121. connection opening;
200. volute fan, 210. air inlet end, 220. exhaust opening;
300. air guide member;
400. air purifying device;
500. base body, 510. bearing platform, 600. induction cooker, 700. induced air pipe.

### DETAILED DESCRIPTION

The embodiments of the present disclosure will be detailed below with examples illustrated in the accompanying drawings. Same or similar numerals represent same or similar elements or elements having same or similar functions throughout the specification. The embodiments described below by referring to the drawings are only illustrative, and used to explain the present disclosure and shall not be understood as limiting of the present disclosure.

The present disclosure relates to a fume extracting mechanism and an induction cooker integrated stove applying the fume extracting mechanism.

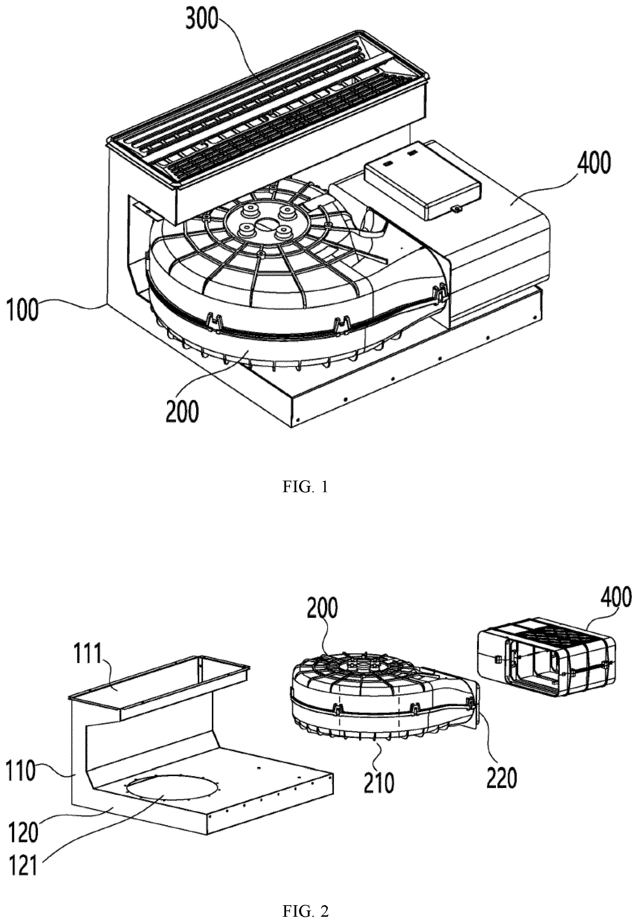

As shown in FIGS. 1 and 2, the fume extracting mechanism includes an air duct assembly 100 and a volute fan 200. The air duct assembly 100 includes a first flow channel 110 and a second flow channel 120, where the first flow channel 110 is in communication with the second flow channel 120. The first flow channel 110 extends along an up-down longitudinal direction and the second flow channel 120 extends along a left-right/back-forth transverse direction. The second flow channel 120 is connected at a lower part of the first flow channel 110. The first flow channel 110 may longitudinally extend along an angle of inclination, and the second flow channel 120 may transversely extend along an angle of inclination. In this embodiment, preferably, the first flow channel 110 is vertically disposed and the second flow channel 120 is horizontally disposed; the second flow channel 120 is connected at a lower end of the first flow channel 110, and the first flow channel 110 and the second flow channel are together shaped like L. The first flow channel 110 is in communication with the second flow channel 120 to form a bending channel inside, and the bending channel is used for air flow. The first flow channel 110 and the second flow channel 120 are both of pipe structure. The first flow channel 110 and the second flow channel 120 may be round pipe structures. In this embodiment, as shown in FIG. 2, the first flow channel 110 and the second flow channel 120 are square pipe structures. The first channel 110 and the second flow channel 120 may be formed by splicing metal plates such as side plates, bottom plates and enclosure plates and the like, but are not limited to the metal plate structure. An air inlet 111 is opened at an upper part of the first flow channel 110 and the air inlet 111 may be opened at a sidewall of the first flow channel 110. Preferably, the air inlet 111 is opened on the top of the first flow channel 110 and the air inlet 111 has an opening facing upward. A connection opening 121 is opened on the second flow channel 120, and the connection opening 121 may be opened at a position such as an end, a sidewall and a bottom and the like of the second flow channel 120. A middle port of the volute fan 200 is used as an air inlet end 210 and a side port is used as an exhaust opening 220. The air inlet end 210 of the volute fan 200 is connected with the connection opening 121 of the second flow channel 120. When the volute fan 200 works, the volute fan 200 performs air suction on the bending channel, such that external air flow enters the air inlet 111 of the first channel 110 and then flows along the bending channel and flows from the second flow channel 120 to the air inlet end 210 of the volute fan 200, and then flows out of the exhaust opening 220 of the volute fan 200. The fume extracting mechanism can be suitable for being mounted at the locations below fume generation, for example, kitchen cabinet and countertop and the like. By using the air duct assembly 100 inverted to perform upward fume suction with the L-shaped bending channel in cooperation with the volute fan 200, on the one hand, the characteristics of high air pressure, low rotation speed, low noise and high energy efficiency can be realized to achieve the effect of noise reduction and air suction improvement, and on the other hand, the entire structure is compact and the occupied space is small, making it more suitable for small mounting spaces.

In some specific embodiments of the present disclosure, the second flow channel 120 horizontally extends with its upper end surface shaped like platform, and the connection opening 121 is opened on the upper end surface of the second flow channel 120. The volute fan 200 is mounted on the upper end surface of the second flow channel 120 such that the second flow channel 120 can bear the volute fan 200. The air inlet end 210 of the volute fan 200 faces downward to connect with the connection opening 121. The exhaust opening 220 of the volute fan 200 faces aside horizontally. This disposal can fully utilize the space above the second flow channel 120 in the air duct assembly 100, making the structure more compact. In this case, no other structural members are needed to fix and support the volute fan 200 separately.

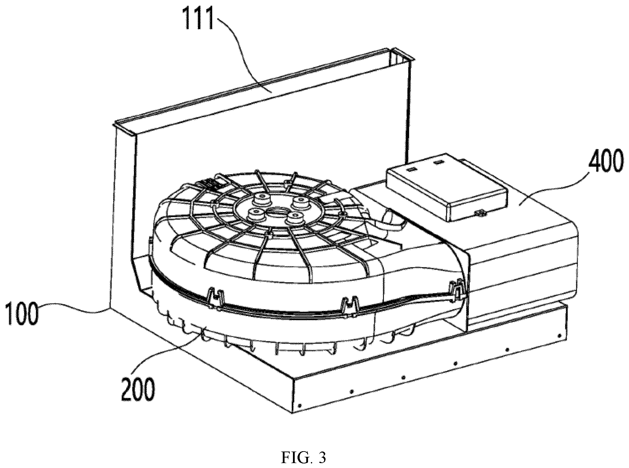

In some specific embodiments of the present disclosure, the air inlet 111 with the opening facing upward is opened on the top of the first flow channel 110. The air inlet 111 may have several sizes, and as shown in FIG. 3, a diameter of the air inlet 111 may be identical to a diameter of the first flow channel 110. Preferably, as shown in FIG. 2, the air inlet 111 is disposed as flaring shape, namely, the diameter of the air inlet 111 is greater than the diameter of the first flow channel 110. When air is conveyed into the first flow channel from the air inlet 111, the flow rate is increased so as to increase a suction force of the air inlet 111. Furthermore, an air guide member 300 is disposed at the air inlet 111, where the air guide member 300 may be a mesh structure and the like. A middle part of the air guide structure protrudes downward, making the entire structure shaped like V. The air guide member 300 has flow guide effect on the fumes while preventing other objects from falling into the first flow channel 110. The air guide member may be directly provided with an oil receiving groove. When fume passes through the air guide member 300, oils in the fume can cling to the air guide member 300, separating from the gas. The volute fan 200 may be a DC variable-frequency speed-regulating system, and can achieve a high level of air pressure of more than 600Pa in cooperation with the air duct assembly 100.

Furthermore, an air purifying device 400 is mounted at the exhaust opening 220 of the volute fan 200. The air purifying device 400 includes an ultraviolet (UV) lamp module and an ionization ozone generation module. An air flow can go through high-voltage ionization sterilization and deodorization treatments through the ionization ozone generation module and receive treatments such as further sterilization, ozone decomposition, and oil drying and the like under the irradiation of the UV lamp module. In this way, the gases to be exhausted can be cleaned, and the surrounding environments can be protected.

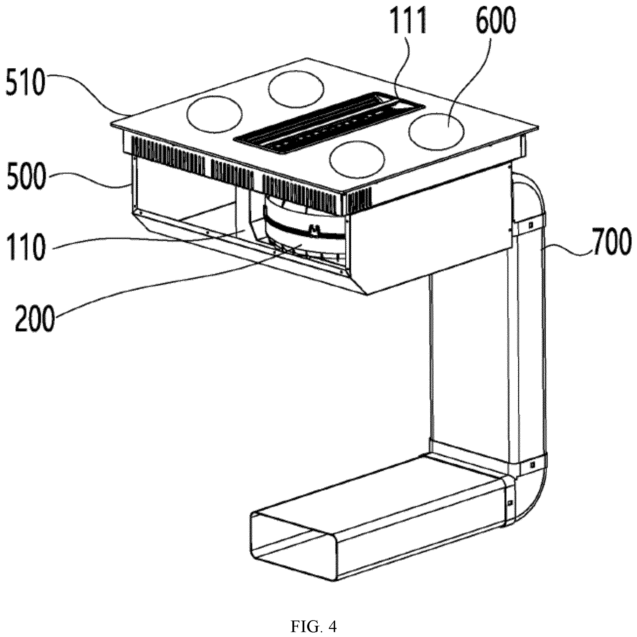

The present disclosure further provides an induction cooker integrated stove, which includes a base body 500, an induction cooker 600 and the above fume extracting mechanism. As shown in FIG. 4, the base body 500 is a frame structure, with its upper part disposed as a bearing platform 510. The bearing platform 510 is used to bear a pan and the like. The induction cooker 600 is mounted on the base body 500. In this embodiment, the induction cooker 600 is located below the bearing platform 510, and the bearing platform 510 can be integrated as an upper plane of the induction cooker 600. The fume extracting mechanism is mounted inside the base body 500, with the first flow channel 110 facing upward. The air inlet 111 is connected to the bearing platform 510. An induced air pipe 700 is disposed on the base body 500. The induced air pipe 700 is in communication with the exhaust opening 220 of the volute fan 200 through an adapter. A length and a direction of the induced air pipe 700 can be determined based on the specific situations of the kitchen cabinet on which the induction cooker integrated stove is mounted.

The pan can be placed on the bearing platform 510 and heated by the induction cooker 600 while the generated fumes can be sucked by the fume extracting mechanism. The induction cooker 600, the fume extracting mechanism and the air purifying device 400 can be integrated together, making the entire volume smaller. During use, the base body 500 can be mounted into the kitchen cabinet in an embedding way. Due to small volume of the fume extracting mechanism, the volume of the entire structure of the induction cooker integrated stove is very small, and thus the stove can adapt to multiple regions of the world and especially to the European region and comply with national standard requirements for kitchen cabinet mounting and placement. In this way, the defect that such products from the fellow companies are used only when the kitchen cabinet mounting size is customized can be overcome and the use requirements of the market users can be satisfied. The air inlet 111 can be disposed as rectangular elongated shape.

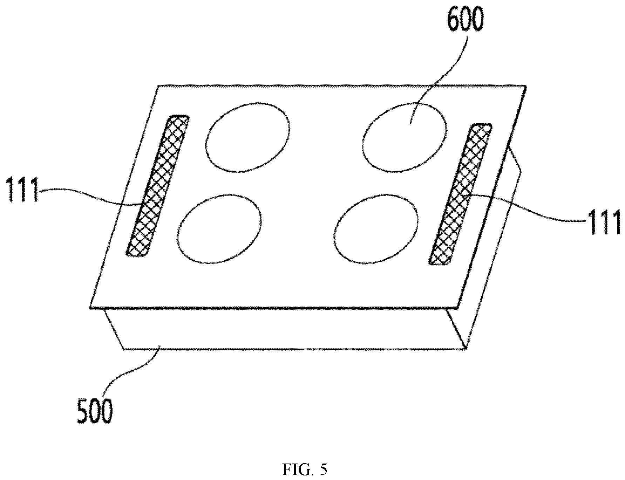

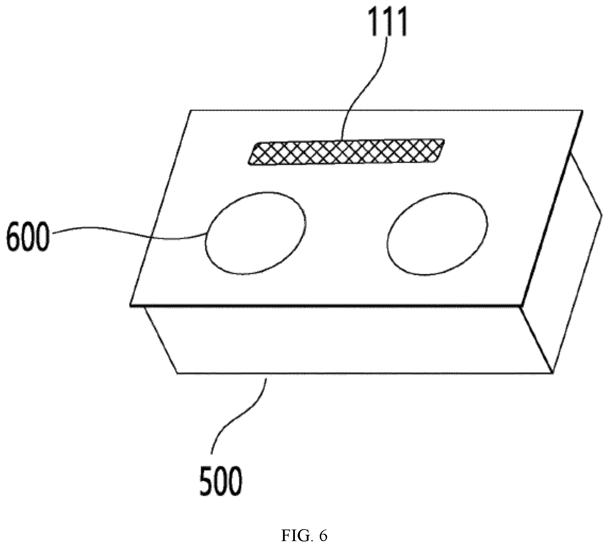

One or more induction cookers 600 may be disposed on the induction cooker integrated stove, and the position of the air inlet 111 may be determined based on the position of each induction cooker 600. As shown in FIG. 4, four rectangularly-distributed induction cookers 600 are disposed, and the air inlet 111 is connected at a middle position of the bearing platform 510. Alternatively, as shown in FIG. 5, two fume extracting mechanisms are disposed oppositely and one air inlet 111 is disposed at both sides of the bearing platform 510 respectively. As shown in FIG. 6, two induction cookers 600 are disposed on the induction cooker integrated stove, and the air inlet 111 can be connected at any of four sides of the bearing platform 510.

The terms such as "first" and "second" are used only for the purpose of descriptions and shall not be understood as indicating or implying any relative importance or implicitly indicating the number of the indicated technical features. Therefore, the features defined by the "first" and "second" may explicitly or implicitly include one or more such features. In the descriptions of the present disclosure, "plural" means at least two, for example, two or three or the like, unless otherwise clearly stated.

In the present disclosure, unless otherwise clearly stated or defined, the terms "mount", "connect", "fix" and the like shall be understood in broad sense, for example, may be fixed connection, or detachable connection, or integral connection; or mechanical connection, or electrical connection; or direct connection or indirect connection through medium or internal communication of two elements or mutual interactions of two elements. Those skilled in the arts may understand the specific meanings of the above terms in the present disclosure based on specific situations.

In the descriptions of the specification, the descriptions made by referring to "some specific embodiments" and the like mean that the specific features, structures, materials or characteristics described by referring to the embodiments or examples are included in any one embodiment or example of the present disclosure. In the specification, the illustrative expressions of the above terms do not necessarily refer to a same embodiment or example. Further, the described specific features, structures, materials or characteristics may be combined in any proper way in any one or more of the embodiments or examples.

Although the embodiments of the present disclosure have been shown and described, persons of ordinary skill in the prior arts may understand that various changes, modifications, replacements and variations can be made to these embodiments without departing from the principle and tenet of the present disclosure, and the scope of protection of the present disclosure shall be defined by claims and its equivalents.

## Claims

1. A fume extracting mechanism, comprising:
an air duct assembly (100), wherein the air duct assembly (100) comprises a longitudinally-extending first flow channel (110) and a transversely-extending second flow channel (120), the second flow channel (120) is in communication with a lower part of the first flow channel (110), an air inlet (111) is opened at an upper part of the first flow channel (110), a connection opening (121) is opened on the second flow channel (120), and the first flow channel (110) and the second flow channel (120) form a bending channel for air flow;
a volute fan (200), wherein an air inlet end (210) in a middle part of the volute fan (200) is connected with the connection opening (121), and the volute fan (200) sucks air from the bending channel such that the air enters the air inlet (111) and flows through the bending channel and then flows out of an exhaust opening (220) of the volute fan (200).

2. The fume extracting mechanism of claim 1, wherein the first flow channel (110) extends vertically, the second flow channel (120) extends horizontally, and the first flow channel (110) and the second flow channel (120) form an L shape.

3. The fume extracting mechanism of claim 1 or 2, wherein the connection opening (121) is opened on an upper end surface of the second flow channel (120), the volute fan (200) is placed on the second flow channel (120), the air inlet end (210) faces downward to connect with the connection opening (121), and the exhaust opening (220) faces horizontally.

4. The fume extracting mechanism of claim 1 or 2, wherein the air inlet (111) with an opening facing upward is opened on a top of the first flow channel (110).

5. The fume extracting mechanism of claim 4, wherein the air inlet (111) is presented as flaring shape, and a diameter of the air inlet (111) is greater than a diameter of the first flow channel (110).

6. The fume extracting mechanism of claim 1, wherein the air inlet (111) is provided with an air guide member (300), and a bottom of the air guide member (300) protrudes downward to form a V shape.

7. The fume extracting mechanism of claim 1, wherein the exhaust opening (220) is provided with an air purifying device (400) which comprises an Ultraviolet (UV) lamp module and an ionization ozone generation module.

8. An induction cooker integrated stove, comprising a base body (500), an induction cooker (600) and the fume extracting mechanism mentioned in any one of claims 1 to 7, wherein the induction cooker (600) is mounted on the base body (500), the fume extracting mechanism is mounted inside the base body (500), an induced air pipe (700) is disposed at a side of the base body (500), the exhaust opening (220) is in communication with the induced air pipe (700), a bearing platform (510) is disposed on an upper part of the base body (500), and the air inlet (111) is connected to the bearing platform (510).

9. The induction cooker integrated stove of claim 8, wherein the air inlet (111) is higher than or flush with an upper end surface of the bearing platform (510), and the induction cooker (600) is located below the bearing platform (510).

10. The induction cooker integrated stove of claim 8 or 9, wherein one or more induction cookers (600) are mounted on the base body (500), and the air inlet (111) is connected at a middle position or a position of a side of the bearing platform (510).
